(19) Europäisches Patentamt
European Patent Office
Office européen des brevets

(11) Veröffentlichungsnummer: **0 039 085**
**A1**

(12) # EUROPÄISCHE PATENTANMELDUNG

(21) Anmeldenummer: **81103230.9**

(22) Anmeldetag: **29.04.81**

(51) Int. Cl.³: **C 07 B 29/00**
C 07 B 27/00, C 07 C 13/62
C 07 D 471/06

(30) Priorität: **30.04.80 DE 3016764**

(43) Veröffentlichungstag der Anmeldung:
**04.11.81 Patentblatt 81/44**

(84) Benannte Vertragsstaaten:
**AT BE CH DE FR GB IT LI NL SE**

(71) Anmelder: **Langhals, Heinz, Dr.**
**Kandelstrasse 15**
**D-7800 Freiburg(DE)**

(72) Erfinder: **Langhals, Heinz, Dr.**
**Kandelstrasse 15**
**D-7800 Freiburg(DE)**

(74) Vertreter: **Kraus, Walter, Dr. et al,**
**Patentanwälte Dres. Kraus & Weisert Irmgardstrasse 15**
**D-8000 München 71(DE)**

(54) **Verfahren zur Erhöhung der Löslichkeit aromatischer Systeme.**

(57) Die Erfindung betrifft ein Verfahren zur Erhöhung der Löslichkeit polycyclischer, homocyclischer oder heterocyclischer, aromatischer Systeme in organischen Lösungsmitteln oder in einer organischen Matrix. Das erfindungsgemäße Verfahren ist dadurch gekennzeichnet, daß man in das polycyclische oder heterocyclische aromatische System eine oder mehrere tert.-Alkylgruppen mit 4 bis 20 Kohlenstoffatomen in an sich bekannter Weise einführt. Bevorzugt verwendet man 1 bis 5 tert.-Alkylgruppen. Bevorzugte tert.-Alkylgruppen sind die tert.-Butylgruppen oder die tert.-Amylgruppen.

EP 0 039 085 A1

2898

Dr. Heinz Langhals

Freiburg

# Verfahren zur Erhöhung der Löslichkeit aromatischer Systeme

## Beschreibung

Die Erfindung betrifft ein Verfahren zur Erhöhung der Löslichkeit polycyclischer, homocyclischer oder heterocyclischer, aromatischer Systeme in organischen Lösungsmitteln oder in einer organischen Matrix.

Die Löslichkeit unsubstituierter und substituierter aromatischer Systeme der homocyclischen oder heterocyclischen Reihe in üblichen organischen Lösungsmitteln sinkt im allgemeinen mit steigender Kondensation und führt bei genügender Anzahl kondensierter Ringe zur völligen Unlöslichkeit in organischen Lösungsmitteln bei Zimmertemperatur (E.Clar, Polycyclic Hydrocarbons, Academic Press, London 1964). Dieser Effekt wird bei den klassischen Küpenfarbstoffen ausgenutzt.

In der Literatur ist die Substitution von Farbstoffen mit t-Butylgruppen unter Steigerung der Löslichkeit in einem Fall beschrieben. Bei Pthalocyaninen konnte eine Steigerung der Löslichkeit erreicht werden (vgl. Geigy, Fr.P. 1,580,683 aus C.A. 73, 100057z (1970) und S.A. mikhalenko, S.V. Barkanova, O.L. Lebeder, E.A. Luk'gants, J.Org.Chem. U.S.S.R. 41, 2770 (1971)).

In der FR-PS 1 580 683 werden lösliche Phthalocyanine beschrieben, die tertiäre Butylgruppen enthalten. Diese Patentschrift bezieht sich ausschließlich auf Phthalocyanine, und es wird in allen Fällen ausdrücklich auf Phthalocyanine hingewiesen, so z.B. in der allgemeinen Formel:

$$Pc-\overset{R_1}{\underset{R_3}{C}}-R_2$$

Dieser Patentschrift läßt sich nur entnehmen, daß Phthalocyanine, die tertiäre Butylgruppen enthalten, extrem löslich in bestimmten organischen Lösungsmitteln sind. Aus dieser Patentschrift ist zu entnehmen, daß es sich hier um eine Besonderheit des Phthalocyanins handelt. Es ist nicht erkannt worden, daß es sich bei der Substitution mit tert-Alkylgruppen um ein universell anwendbares Prinzip zur Erhöhung der Löslichkeit von aromatischen Systemen handelt. In der französischen Patentschrift werden als Lösungsmittel nur aliphatische und aromatische Kohlenwasserstoffe, Pyridin und chlorierte Kohlenwasserstoffe angegeben. Allgemeine Hinweise, wie die Löslichkeit höher kondensierter aromatischer Systeme in üblichen organischen Lösungsmitteln erhöht werden kann, lassen sich dieser Literaturstelle nicht entnehmen.

Die schlechte Löslichkeit höher kondensierter aromatischer Systeme in üblichen organischen Lösungsmitteln ist nachteilig und hindert die Verwendung solcher Verbindungen, z.B. als Farbstoffe zum homogenen Einfärben organischer Medien oder als Monomere, die dadurch nicht für Polymerisationsreaktionen eingesetzt werden konnten, zur Gewinnung Polymerer mit bisher nicht bekannten Eigenschaften, erschwert ihre Darstellung und Reinigung und erhöht ihre Herstellungskosten. Insbesondere bei Farbstoffen ist die schlechte Löslichkeit höher kondensierter Verbindungen von Nachteil, da dadurch ihre Anwendung erschwert und in vielen Fällen praktisch unmöglich wird.

Der vorliegenden Erfindung liegt die Aufgabe zugrunde, ein Verfahren zur Verfügung zu stellen, mit dem die Löslichkeit höher kondensierter aromatischer Systeme, die auch Heteroatome enthalten können, in organischen Lösungsmitteln und in Matrices erhöht werden kann, ohne daß sich die charakteristischen Eigenschaften der aromatischen Systeme ändern.

Gegenstand der Erfindung ist somit ein Verfahren zur Erhöhung der Löslichkeit polycyclischer, homocyclischer oder heterocyclischer, aromatischer Systeme in organischen Lösungsmitteln oder einer organischen Matrix, das dadurch gekennzeichnet ist, daß man in das polycyclische oder heterocyclische, aromatische System eine oder mehrere tert.-Alkylgruppen mit 4 bis 20 Kohlenstoffatomen in an sich bekannter Weise einführt.

Überraschenderweise wurde gefunden, daß sich die Löslichkeit höher kondensierter aromatischer Systeme durch die Einführung von tert.-Alkylgruppen in organischen Lösungsmitteln und Matrices wesentlich erhöht.

Das erfindungsgemäße Verfahren besitzt somit die folgenden Vorteile:

(1) Starke Erhöhung der Löslichkeit des substituierten Systems;

(2) keine Verringerung der thermischen Stabilität;

(3) keine Verringerung der Photostabilität;

(4) keine leicht ablaufende Oxidationsvorgänge oder verwandte Reaktionen (nur primäre Wasserstoffatome, keine Benzylstellungen);

(5) keine Quenchvorgänge bei fluoreszierenden Systemen;

(6) Verringerung von Assoziatbildung;

(7) leichte Durchführbarkeit durch Friedel-Crafts-Alkylierung oder nukleophile Substitution.

Bei dem erfindungsgemäßen Verfahren können als polycyclische, homocyclische oder heterocyclische, aromatische Systeme homocyclische Kohlenwasserstoffe, wie Naphthalin, Anthracen, Phenanthren, Chrysen sowie 1,2-Benzanthracen, verwendet werden.

Bei dem erfindungsgemäßen Verfahren können weiterhin heterocyclische, aromatische Systeme als Ausgangsmaterial verwendet werden. Diese Heterocyclen können mit einem oder mehreren Heteroatomen, beispielsweise N, O, S, substituiert sein. Beispiele hierfür sind: Indol, Indozilin, Cumaron, Thionaphthen, Triphenylen; Decacyclen; 3,4-Benzophenanthren; 1,2-Benzochrysen; Picen; 5,6-Benzochrysen; 3,4,5,6-Dibenzophenanthren; Hexahelicen; 1,2,7,8-Dibenzochrysen; 11,12,13,14-Dibenzopicen; 1,2,3,4,5,6,7,8,9,10,11,12-Hexabenzotriphenylen; Anthracen; Tetraphen; 1,2,3,4-Dibenzanthracen; 1,2,5,6-Dibenzanthracen; 1,2,7,8-Dibenzanthracen; 1,2,3,4,5,6-Tribenzanthracen; 1,2,3,4,5,6,7,8-Tetrabenzanthracen; 1,2-Benzotetraphen; 3,4-Benzotetraphen; 3,4,8,9-Dibenzotetraphen; Pentaphen; 3,4-Benzopentaphen; 3,4,9,10-Dibenzopentaphen; 1,2,5,6-Dibenzotetraphen; 6,7-Benzopentaphen; Dinaphtho-(2',1':1,2);-(2",1":5,6)-anthracen; Naphtho-(2',3':6,7)-pentaphen; Naphtho-(2',3':3,4)-pentaphen; Anthraceno-(2',1':1,2)-anthracen; Anthraceno-(2',1':8,9)-tetraphen; Tetrapheno-(9',8':8,9)-tetraphen; 2,3-Benzopicen; 2,3,8,9-Dibenzopicen; Anthraceno-(1',2':1,2)-tetraphen; Tetracen; 1,2-Benzotetracen; 1,2,3,4-Dibenzotetracen; 1,2,7,8-Dibenzotetracen; 1,2,9,10-Dibenzotetracen; 1,2,3,4,7,8-Tribenzotetracen; 1,2,3,4,7,8,9,10-Tetrabenzotetracen; Hexaphen; Heptaphen; 7,8-Benzoheptaphen; Pentacen; 1,2-Benzopentacen; 1,2,3,4-Dibenzopentacen; 1,2,8,9-Dibenzopentacen; 1,2,10,11-Dibenzopentacen; 1,2,3,4,8,9,10,11-Tetrabenzopentacen; Hexacen; 1,2-Benzohexacen; Heptacen; Octacen; Nonacen; Undecacen; Diphenyl; Fluoren; Biphenylen;1,2-Benzobiphenylen; 2,3-Benzobiphenylen; 1,2,5,6-Dibenzoperylen; 1,2,5,6,7,8,11,12-Tetrabenzoperylen; 2,3,8,9-Dibenzoperylen; 2,3,10,11-Dibenzoperylen; Dinaphtho-(2',3':2,3);(2",3":8,9)-perylen; 1,12-Benzoperylen; 1,12,2,3-Dibenzoperylen; 1,12,2,3,8,9-Tribenzoperylen; 1,12,2,3,10,11-Tribenzoperylen; 1,12,4,5,8,9-Tribenzoperylen; 1,12,5,6,7,8-Tribenzoperylen; 1,12-o-Phenylenperylen; Anthraceno-(1',4':1,12)-perylen; 1,12-o-Phenylen-2,3-benzoperylen; 1,12-o-Phenylen-2,3,10,11-dibenzoperylen; 1,2,3,4,5,6,10,11-Tetrabenzanthanthen; Coronen; 1,2-Benzocoronen; 1,2,5,6-Dibenzocoronen; 1,2,7,8-Dibenzocoronen; 1,2,3,4,5,6-Tribenzocoronen; Naphtho-(2',3':1,2)-coronen; 1,12,2,3,4,5,6,7,8,9,10,11-Hexabenzocoronen; Bisanthen; 1,14-Benzobisanthen; Ovalen; 3,4,10,11-Dibenzobisanthen; 3,4,11,12-Dibenzobisanthen; 1,2,3,4,8,9,10,11-Tetrabenzobisanthen; Circumanthracen; Pyren; 1,2-Benzopyren; 3,4-Benzopyren; 1,2,3,4-Dibenzopyren; Perylen; 1,2-Benzoperylen; 2,3-Benzoperylen; 1,2,7,8-Dibenzoperylen, 1,2,10,11-Dibenzoperylen; 1,2,11,12-Dibenzoperylen; 1,2,4,5-Dibenzopyren; 1,2,6,7-Dibenzopyren; 3,4,8,9-Dibenzopyren; 3,4,9,10-Dibenzopyren; 1,2,4,5,8,9-Tribenzopyren; 1,2,3,4,9,10-Tribenzopyren; 1,2,3,4,6,7,8,9-Tetrabenzopyren; Naphtho-(2',3':1,2)-pyren; Naphtho-(1',2':3,4)-pyren; Naphtho-(2',3':3,4)-pyren; 1,2-Benzonaphtho-(2",3":4,5)-pyren; 1,2-Benzonaphtho-(2",3":6,7)-pyren; 8,9-Benzonaphtho-(2",1":3,4)-pyren; 3,4-Benzanthraceno-(2",1':8,9)-pyren; 3,4-Benzonaphtho-(2",3":9,10)-pyren; Dinaphtho-(2',3':1,2);(2",3":4,5)-pyren; Dinaphtho-(2',3':1,2);(2",3":6,7)-pyren; Dinaphtho-(2',3':3,4); (2",3":9,10)-pyren; 1,2-Benzodinaphtho-(2",3":4,5); (2",3":8,9)-pyren; 1,14,4,5-Dibenzopentacen; Naphtho-(1',7':2,14)-pentacen; Phenanthreno-(2',3':3,4)-pyren; 1,2-Benzophenanthreno-(9',10':6,7)-pyren; 1,2,3,4,6,7,12,13-Tetrabenzopentacen; 1,16,4,5-Dibenzohexacen; 5,6,15,16-Dibenzohexacen; Naphtho-(1',7':2,16)-hexacen; 6,7,16,17-Dibenzoheptacen; 5,6,8,9,14,15,17,18-Tetrabenzoheptacen, 1,18,4,5,9,10,13,14-Tetrabenzoheptacen; Dinaphtho-(1',7':2,18) ;(7",1":9,11)-heptacen; 7,8,17,18-Dibenzoctacen; Anthanthren; 1,2,7,8-Dibenzoanthanthren; 2,3,4,5-Dibenzanthanthren; 2,3,8,9-Dibenzanthanthren; 1,2-Benzonaphtho-(2",1":7,8)-anthanthren; Dinaphtho-(2',1:1,2):(2",1":7,8)-anthanthren; Dinaphtho-(1',2':1,2);(1",2":7,8)-anthanthren; 1,2-Benzophenanthreno-(10",2":8,10)-anthanthren; p-Terphenyl; 1,9,5,10-Di(peri-naphthylen)-anthracen; Terrylen; 7,8-Benzoterrylen; 1,2,13,14-Dibenzoterrylen; 7,8,15,16-Dibenzoterrylen; 2,3-peri-Naphthylenpyren; Pyreno-(1,3:10',2')-pyren; 1,12,2,3,6,7,8,9-Tetrabenzanthanthren; 9,10,3',4'-Dibenzopyreno-(1,3:10',2')-pyren; Peropyren; 1,2,8,9-Dibenzoperopyren; 1,2,9,10-Dibenzoperopyren; 1,2,6,7-Dibenzoperopyren; 1,2,11,12-Dibenzoperopyren; 4,5,11,12-Dibenzoperopyren; 4,5,6,7,11,12,13,14-Tetrabenzoperopyren; 5,6,12,13-Dibenzoperopyren; 2,3,5,6,8,9-Tribenzoperopyren; 1,14,7,8-Dibenzoperopyren; 1,14,10,11-Dibenzoperopyren; 3,4,5,6,7,8-Tribenzoperopyren; 3,4,5,6,10,11,12,13-Tetrabenzoperopyren; Dinaphtho-(7',1':1,13);(1",7":6,8)-peropyren; Dinaphtho-(7',1':1,13);(1",7":9,11)-peropyren; Dinaphtho-(1',7':2,4);(1",7":9,11)-peropyren; Dinaphtho-(1',7':2,4);(2",8":7,9)-peropyren; Dinaphthoperopyrene; Quaterrylen; 1,20,16,17-Dibenzoquaterrylen; 2,3,7,8-Di-(peri-naphthylen)-pyren; Dipyreno-(1',3':10,2);(1",3":5,7)-pyren; Quinquiphenyl; Sexiphenyl; Fluoranthen; 2,3-Benzofluoranthen; 2,3,6,7-Dibenzofluoranthen; 3,4-Benzofluoranthen; 10,11-Benzofluoranthen; 11,12-Benzofluoranthen; 3,4,11,12-Dibenzofluoranthen; 9,10,11,12-Dibenzofluoranthen; 2,3,6,7,10,11-Tetrabenzofluoranthen; Naphtho-(1',2':2,3)-fluoranthen; Naphtho-(2',1':2,3)-fluoranthen; Naphtho-(2',3':3,4)-fluoranthen; Naphtho-(2',3':10,11)-fluoranthen; Naphtho-(2',3':11,12)-fluoranthen; 10,11-Benzonaphtho-(2",3",3,4)-fluoranthen; 2,3-Benzonaphthofluoranthene; 2,3-o-Phenylenpyren; 2,3-o-Phenylen-4,5-benzopyren; 2,3-o-Phenylennaphtho-(2",3":4,5)-pyren; 4",5-o-Phenylennaphtho-(2",3":3,4)-pyren; 2,13-Benzofluoranthen; 2,13-o-Phenylenfluoranthen; 2,13,11,12-Dibenzofluoranthen; 4,5-o-Phenylenfluoranthen; Isorubicen; 5,6,11,12-D-(o-Phenylen)-tetracen; Rubicen; 2,3-Benzorubicen; Dibenzorubicene; 2,3,7,8-Di-(o-phenylen)-pyren; Periflanthen; 1,16-Benzoperiflanthen; 10,11-peri-Naphthylenfluoranthen; 11,12-peri-Naphthylenfluoranthen; 2,3,6,7-Di-(peri-naphthylen)-naphthalin; 1,2,3,4-Di-(peri-naphthylen)-anthracen; 2,3,6,7-Di-(peri-naphthylen)-anthracen; Di-(o-phenylen)-phenanthren; 2,3-o-Phenylen-4,5-peri-naphthylenpyren; Decacylen; 1,2,3,4,5,6,7,8-Tetra-(peri-naphthylen)-anthracen; Perinaphthen; Benzanthrene; Naphtho-(2',1':7,8)-perinaphthen; Naphtho-(1',2':7,8)-perinaphthen; 2,3,7,8-Dibenzoperinaphthen; Naphtho-(2',3':7,8)-perinaphthen; 4,5,7,8-Dibenzoperinaphthen; Coeranthren; Naphthanthren; 3H-4,5-Benzonaphthanthren; 3,4-Benzonaphthanthren; 1,2,10,11-Dibenzonaphthanthren; 2,3-Benzonaphtho-(2",3":7,8)-perinaphthen; 7,8-Benzonaphtho-(2",3":4,5)-peripnaphthen; 1,2,4,5,7,8-Tribenzoperinaphthen; 7,8-Benzonaphtho-(1",2":4,5)-perinaphthen; Anthraceno-(2',3':7,8)-perinaphthen; 7,8-Benzanthraceno-(2",3":4,5)-perinaphthen; 4,8-Dihydrotriangulen; 5,6-Dihydro-1,12,10,11-dibenzotetracen; 3,11-Dihydro-1,2,4,5,6,7-tribenzotetracen; 5,7-Dihydro-1,14,11,12-dibenzopentacen; Zethren; 5,6-Benzozethren; 4,5,11,12-Dibenzozethren; 5,6,12,13-Dibenzozethren; Heptazethren; 4,5,12,13-Dibenzoheptazethren; 5,6,13,14-Dibenzoheptazethren; Indeno-(2',1':1,2)-perinaphthen; 1,2,4,5-Dibenzopentalen, Dianthraceno-(1',9':1,3);(1",9":4,6)-pentalen; Carbazol; Diphenylenoxid; Diphenylensulfid; Indazol; Oxazol; Isoxazol; Thiazol; Isothiazol; Benzthiazol; Triazole; Pyridin; Chinolin; Isochinolin; Acridin; Phenanthridin; Pyridazin; Pyrimidin; Pyrazin; Cinnolin;Benzo[c]cinnolin; Phthalazin; Chinazolin; Chinoxalin; Phenazin; Phenoxazin; Phenothiazin und Thianthren.

Grundsätzlich kann man alle die Kohlenwasserstoffe als Ausgangsmaterialien verwenden, die in dem zuvor erwähnten Buch von E. Clar aufgeführt werden.

In den obengenannten Kohlenwasserstoff-Systemen sind besonders bevorzugt Naphthalin, Anthracen, Phenanthren, Chrysen, 1,2-Benzanthracen, Decacyclen, Hexahelicen, Tetracen, Diphenyl, Fluoren, 1,12-Benzoperylen, 1,12-o-Phenylenperylen, Coronen, 1,2-Benzocoronen, Bisanthen, 1,4-Benzobisanthen, Ovalen, Circumanthracen, Pyren, Perylen, 1,2,8,9-Dibenzoperopyren, 1,2,9,10-Dibenzoperopyren, Dipyreno-(1',3':10,2);(1",3":5,7)-pyren, Fluoranthen, 2,3-o-Phenylenpyren, 4,5-o-Phenylenfluoranthen, Isorubicen, Rubicen, 2,3,7,8-Di-(o-phenylen)-pyren, Periflanthen, 11,12-peri-Naphthylenfluoranthen, 2,3,6,7-Di-(peri-naphthylen)-naphthalin, 2,3-o-Phenylen-4,5-perinaphthylenpyren, Decacylen, 1,2,3,4,5,6,7,8-Tetra-(peri)-naphthylen)-anthracen, Indeno-(2',1':1,2)-perinaphthen und 1,2,4,5-Dibenzopentalen.

Die obengenannten homocyclischen oder heterocyclischen Systeme können weiterhin substituiert sein. Die Zahl der Substituenten ist nicht kritisch. Beispielsweise können die Verbindungen durch 1 bis 5, vorzugsweise 1 bis 3 Substituenten substituiert sein. Beispiele von Substituenten

sind Alkylgruppen mit 1 bis 10 und vorzugsweise 1 bis 5 C-Atomen, Alkenylgruppen mit 2 bis 8 und vorzugsweise 2 bis 6 C-Atomen, Hydroxygruppen, Carboxygruppen, Halogenatome (Fluor, Chlor, Brom oder Jod), Trifluormethylgruppen, Äthergruppen mit 1 bis 10 und vorzugsweise 1 bis 6 C-Atomen, Cyanogruppen, Säureamidgruppen, Sulfonsäuregruppen, Sulfone, Sulfonsäureamidgruppen mit 0 bis 10 und vorzugsweise 1 bis 6 C-Atomen, Carbonsäureestergruppen, Mercaptogruppen, Thioäthergruppen mit 1 bis 10 C-Atomen, Aminogruppen, N-Alkylaminogruppen mit 1 bis 10 und vorzugsweise 1 bis 6 C-Atomen, N,N-Dialkylaminogruppen mit 2 bis 10 und vorzugsweise 2 bis 6 C-Atomen, phosphorhaltige Gruppen, z.B.Phosphorsäureester, usw..

Das erfindungsgemäße Verfahren eignet sich besonders gut für die Solubilisierung organischer Farbstoffe.

Beispiele von Farbstoffen, die durch das erfindungsgemäße Verfahren in lösliche Formen überführt werden können, sind:

1) Nitrofarbstoffe
2) Nitrosofarbstoffe
3) Diphenylmethanfarbstoffe
4) Triphenylmethanfarbstoffe
5) Acridinfarbstoffe
6) Xanthenfarbstoffe
7) Azinfarbstoffe
8) Methinfarbstoffe
9) Chinonimine
10) Anthrachinonfarbstoffe
11) Indigoide Farbstoffe
12) Chinoide Küpenfarbstoffe
13) Indigosole
14) Schwefelfarbstoffe
15) Stilbenfarbstoffe
16) Thiazolfarbstoffe
17) Azofarbstoffe
18) Porphyrine

oder deren Vorstufen.

Bei dem erfindungsgemäßen Verfahren werden in die in organischen Lösungsmitteln schwer löslichen Verbindungen eine oder mehrere tert.-Alkylgruppen eingeführt. Im allgemeinen werden 1 bis 5 und besonders bevorzugt 1 bis 3 und 1 bis 2 tert.-Alkylgruppen eingeführt. Die Zahl der tert.-

Alkylgruppen spielt keine wesentliche Rolle. Es hat sich gezeigt, daß schon durch die Einführung einer tert.-Alkylgruppe die Löslichkeit in organischen Lösungsmitteln wesentlich gesteigert wird. Die Zahl der einzuführenden tert.-Alkylgruppen hängt auch in gewissem Ausmaß von der Zahl der kondensierten aromatischen Ringe ab und davon, wieviel Heteroatome in der Ausgangsverbindung vorhanden sind. Im allgemeinen reicht die Einführung von 1 bis 3 tert.-Alkylgruppen zur Erhöhung der Löslichkeit aus. Es ist jedoch auch möglich, mehrere tert.-Alkylgruppen einzuführen. Die Zahl der einzuführenden tert.-Alkylgruppen hängt weiterhin von sterischen Faktoren ab.

Die tert.-Alkylgruppen können 1 bis 20 Kohlenstoffatome enthalten. Vorzugsweise enthalten sie jedoch 4 bis 10, besonders bevorzugt 4 bis 8 und am meisten bevorzugt 4 oder 5 Kohlenstoffatome.

Am meisten bevorzugt sind somit die tert.-Butylgruppe und die tert.-Amylgruppe. Die tert.-Butylgruppe kann leicht eingeführt werden und das Ausgangsmaterial zur Einführung der tert.-Butylgruppe ist leicht erhältlich.

Die Einführung der Alkylgruppen, insbesondere der tert.-Butylgruppe, erfolgt durch übliche Alkylierung, beispielsweise durch Friedel-Crafts-Alkylierung ("Friedel-Crafts and Related Reactions", G.A. Olah, Interscience Publishers John Wiley, New York 1963, insbes. Bd. 2 und die dort genannten Literaturstellen) oder nukleophile Substitution (vgl. H.E. Ziegler, J.E. Rosenkranz, J.Org.Chem. 29, 2469, 1964 und die dort genannten Literaturstellen).

Bei der Friedel-Crafts-Alkylierung kann man alle üblichen Friedel-Crafts-Katalysatoren verwenden. Beispiele hierfür sind Lewissäuren, wie Aluminiumchlorid, Bortrifluorid, Chlorwasserstoffsäure, Phosphorsäure und $FeCl_3$, Zinkchlorid, Schwefelsäure, Flußsäure, Antimonpentachlorid, Titantetrachlorid, Zinntetrachlorid, Bortrichlorid und Aluminiumbromid.

Normalerweise erfolgt die Durchführung der Friedel-Crafts-Alkylierung, indem man die zu alkylierende Verbindung in einem inerten, wasserfreien Lösungsmittel suspendiert und das Alkylierungsmittel und den Friedel-Crafts-Katalysator zugibt, anschließend gegebenenfalls erwärmt und unter Wasserausschluß das Reaktionsgemisch rührt. Das Erhitzen kann auf eine Temperatur von Zimmertemperatur bis zur Siedetemperatur des Lösungsmittels erfolgen. Verwendet man beispielsweise Schwefelkohlenstoff als Suspensionsmittel, so erhitzt man auf 30 bis 40°C. Die Reaktionszeit beträgt 2 bis 20 Stunden, vorzugsweise 5 bis 15 Stunden, und hängt von der verwendeten Temperatur und dem Ausgangsmaterial ab.

Als Alkylierungsmittel verwendet man die entsprechenden Halogenide oder Olefine der einzuführenden Alkylgruppen, beispielsweise die Chloride oder die Bromide. Bei der Einführung von einer tert.-Butylgruppe verwendet man beispielsweise Butylchlorid, Butylbromid oder Isobuten.

Die Aufarbeitung des Reaktionsgemisches erfolgt in an sich bekannter Weise. Im allgemeinen gibt man zu dem Reaktionsgemisch Wasser, destilliert das Lösungsmittel ab und extrahiert den erhaltenen Rückstand mit einem geeigneten Lösungsmittel.

Geeignete Lösungsmittel sind beispielsweise Methylenchlorid, Chloroform, Toluol, und Äthylacetat.

Die vereinigten Methylenchloridextrakte werden dann in an sich bekannter Weise gereinigt, das Lösungsmittel wird abdestilliert und der erhaltene Rückstand gereinigt.

Die Einführung der tert.-Alkylgruppen kann auch durch nukleophile Substitution erfolgen. Besonders geeignet ist

die Umsetzung elektronenarmer Aromaten, wie z.B.Pyridin, Acridin, Chinoline, Benzo[c]cinnolin, Phenanthroline, Pyryliumsalze, Azulene, Tropyliumsalze oder Perylen, mit metallorganischen Reagentien, wie z.B. tert.-Butyllithium oder tert.-Amyllithium, in verschiedenen inerten Lösungsmitteln, wie z.B. Äther, THF, Dioxan.

Der Einfluß der Substitution mit tert.-Butylgruppen wurde an folgenden Systemen untersucht.

(1) Rubicen (I)
Das Rubicen wurde bei Friedel-Crafts-Bedingungen gemäß folgender Gleichung zu Di-tert.-butylrubicen (II) alkyliert:

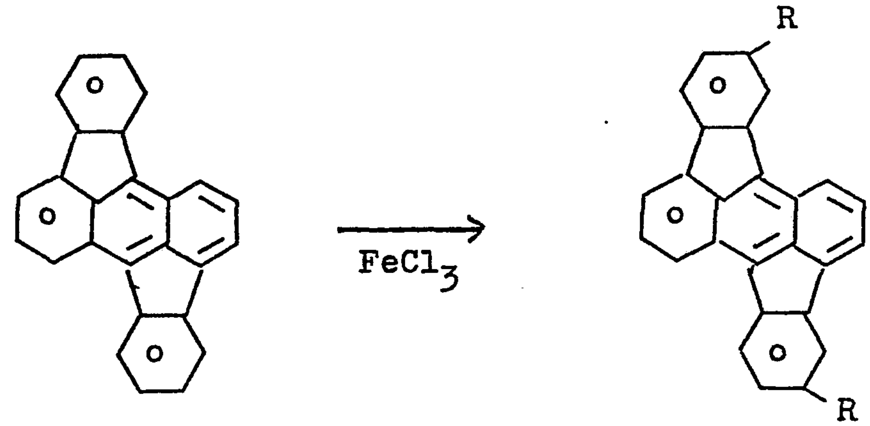


I II

Die zweifache tert.-Butylierung brachte eine Löslichkeitssteigerung um einen Faktor von ca. 100 in Äthanol und n-Hexan bei Zimmertemperatur. Die vierfache tert.-Butylierung zu tetra-tert.-Butylrubicen mit unbekannter Lage der Substituenten erhöhte die Löslichkeit um einen Faktor von ca. $10^4$.

(2) Perylenfarbstoffe (III)
Der Perylenfarbstoff der Formel III

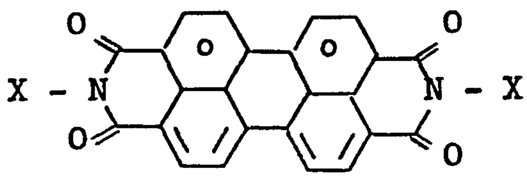

III

worin X Phenyl bedeutet, weisen in organischen Lösungsmitteln eine sehr geringe Löslichkeit auf. Durch Ersatz von X durch 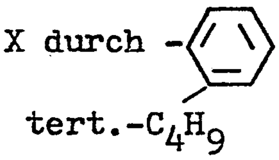 wird die Löslichkeit in organischen Lösungsmitteln allgemein um einen Faktor von allgemein ca. $10^3$ erhöht.

In allen untersuchten Fällen wird die Photostabilität und die Fluoreszenzquantenausbeute der Verbindungen nicht nachteilig beeinflußt.

Die folgenden Beispiele erläutern die Erfindung.

## Beispiel 1

## Di-tert.-butylrubicen (II; R = tert.-$C_4H_9$)

1 g (3 mMol) Rubicen wird in 100 ml wasserfreiem Schwefelkohlenstoff suspendiert und mit 20 ml tert.-Butylchlorid und 50 mg wasserfreiem Eisen(III)-chlorid versetzt. Anschließend wird unter Rühren und Wasserausschluß 12 h lang auf 40°C erhitzt. Das Reaktionsgemisch wird mit 200 ml Wasser versetzt und der Schwefelkohlenstoff wird danach abdestilliert. Der Rückstand wird dreimal mit Methylenchlorid extrahiert.

Die vereinigten Methylenchloridextrakte werden dreimal mit 2 m Salzsäure, einmal mit 2 m Natriumcarbonatlösung und dreimal mit Wasser ausgeschüttelt. Das Lösungsmittel wird

abgedampft und der Rückstand mit n-Hexan über eine Säule mit Kieselgel (MN Kieselgel 60 0,063-0,2 mm der Firma Macherey-Nagel) filtriert. Zur weiteren Reinigung wird noch zweimal mit Tetrachlorkohlenstoff über eine Säule mit Kieselgel chromatographiert. Das Reaktionsprodukt ist dünnschichtchromatographisch einheitlich.

Ausbeute: 60 mg (4,5%) $R_f$: 0,39 ($CCl_4$/Kieselgel).

Beispiel 2
Tetra-tert.-butylrubicen

1 g (3 mMol) Rubicen wird in 100 ml wasserfreiem Schwefelkohlenstoff vorgelegt und mit 10 ml tert.-Butylchlorid und 50 mg wasserfreiem Aluminiumchlorid versetzt. Anschließend wird unter Rühren und Feuchtigkeitsausschluß 4 h auf 30 bis 40°C erhitzt.

Das Reaktionsgemisch wird mit 200 ml Wasser versetzt und der Schwefelkohlenstoff wird anschließend abdestilliert. Der Rückstand wird dreimal mit Methylenchlorid extrahiert. Die vereinigten Extrakte werden mit 2 m Natriumcarbonatlösung und dreimal mit Wasser gewaschen. Das Methylenchlorid wird abdestilliert und der Rückstand mit Hexan über eine Säule mit Kieselgel filtriert. Das Hexan wird abgedampft und der Rückstand zweimal mit einem Laufmittelgemisch Essigester/Hexan (1:4) über eine Säule mit Kieselgel chromatographiert. Das erhaltene Produkt ist dünnschichtchromatographisch einheitlich.

Ausbeute: 120 mg (7%).

Beispiel 3

Darstellung von "Perterbutan" (III; X = 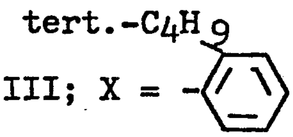 )

N,N'-Di-(2-tert.-butyl)-perylen-3,4,9,10-tetracarbonsäurediimid

1 g (2,5 mMol) Perylen-3,4,9,10-tetracarbonsäuredianhydrid wird mit 350 mg Zinkacetat, 5 ml Chinolin und 1,5 g (10 mMol) o-tert.-Butylanilin vermengt und 2 h auf 220 bis 230°C unter Reinstickstoff erhitzt. Nach dem Abkühlen wird der Feststoff abgesaugt und mit kleinen Portionen warmem Äthanol so lange gewaschen, bis der Chinolingeruch verschwunden ist.

Rohausbeute: 1,1 g (73%).

Das Produkt besitzt nach IR-Spektrum und Dünnschichtchromatographie eine Reinheit von mehr als 85%.

Zur weiteren Reinigung werden 850 mg Farbstoff-Rohprodukt in 800 ml heißem Toluol gelöst. Nach dem Erkalten werden 200 ml Aceton zugesetzt und vom Ungelösten (450 mg) abfiltriert. Die Lösung wird anschließend über neutrales Aluminiumoxid filtriert (Säulendurchmesser 4,5 cm, Aluminiumoxid-Füllung 15 cm hoch), wobei nichtumgesetztes Dianhydrid quantitativ zurückgehalten wird. Nach dem Abdestillieren des Elutionsmittels Toluol erhält man 220 mg reinen Farbstoff in Form eines leuchtendroten Pulvers.

IR: Imid 1705, $CH_3$ 2960, 2910, 2870 $cm^{-1}$

DC ($CHCl_3$, $Al_2O_3$ neutral): $R_f = 0,64$

$C_{44}H_{34}N_2O_4$:

| | | | |
|---|---|---|---|
| berechnet: | C 80,71% | H 5,23% | N 4,28% |
| gefunden : | 79,84 | 5,30 | 4,91 |

Fluoreszenzquantenausbeute (absolut):
$\eta$ = 1,0 (Chloroform)
$\eta$ = 0,94 (Dimethylformamid)

UV (DMF): 524 (4,86), 488 (4,67), 458 (4,24) nm
Fl (DMF): 538, 573 nm.

Beispiel 4
Darstellung von "Paraterbutan" (X = 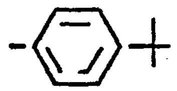 )

N,N'-Di-(4-tert.-butylphenyl)-perylen-3,4,9,10-tetracarbonsäurediimid

Die Darstellung erfolgt auf gleiche Weise wie in Beispiel 3 beschrieben.

Rohausbeute: 98% Reinheit (IR, DC): > 95%
Reinigung: Filtration einer Lösung des Farbstoffs über neutr. $Al_2O_3$ mit DMF
Farbe des Reinproduktes: dunkelweinrot
Schmelzpunkt: > 500°C
IR (KBr)($cm^{-1}$): Imid 1710, 1670; $CH_3$ 2960, 2900, 2860
DC ($CHCl_3$, $Al_2O_3$ neutral): $R_f$ = 0,70
Analyse: $C_{44}H_{34}N_2O_4$

| | | | |
|---|---|---|---|
| berechnet: | C | H | N |
| gefunden : | | | |

Fluoreszenzquantenausbeute (absolut):
$\eta$ = 0,88 (DMF)

UV (DMF) (nm) (log $\varepsilon$): 522 (4,38), 487 (4,22), 456 (3,83)
Fluoreszenz (DMF): 573, 536
Massenspektrum (m/e): 654 ($M^+$), I. 59
639 ($-CH_3$), I. 100.

Beispiel 5

Darstellung von "Perditer" (X = 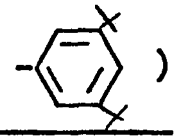 )

N,N'-Di-(3,5-di-tert.-butylphenyl)-perylen-3,4,9,10-tetracarbonsäurediimid

Die Darstellung erfolgt auf gleiche Weise wie in Beispiel 3 beschrieben.

Rohausbeute: 84% Reinheit (IR, DC): > 85%

Reinigung: Filtration einer Lösung des Farbstoffs über neutr. $Al_2O_3$ mit Toluol/Aceton (9:1).

Farbe des Reinproduktes: rot - rotbraun

Schmelzpunkt: > 300°C

IR (KBr)($cm^{-1}$): Imid 1705, 1665; $CH_3$ 2960, 2900, 2860

DC ($CHCl_3$, $SiO_2$): $R_f$ = 0,32

Analyse: $C_{52}H_{50}N_2O_4$

berechnet: C H N

gefunden :

Fluoreszenzquantenausbeute (absolut):

$\eta$ = (DMF)

UV (DMF)(nm)(log $\varepsilon$): 524 (4,88), 488 (4,68), 458 (4,25)

Fluoreszenz (DMF)(nm): 575, 537

Massenspektrum (m/e):

0039085

Patentansprüche

1. Verfahren zur Erhöhung der Löslichkeit polycyclischer, homocyclischer oder heterocyclischer, aromatischer Systeme in organischen Lösungsmitteln oder in einer organischen Matrix, dadurch gekennzeichnet, daß man in das polycyclische oder heterocyclische aromatische System eine oder mehrere tert.-Alkylgruppen mit 4 bis 20 Kohlenstoffatomen in an sich bekannter Weise einführt.

2. Verfahren nach Anspruch 1, dadurch gekennzeichnet, daß man 1 bis 5 tert.-Alkylgruppen einführt.

3. Verfahren nach einem der Ansprüche 1 bis 2, dadurch gekennzeichnet, daß man tert.-Butylgruppen oder tert.-Amylgruppen einführt.

4. Verfahren nach einem der vorhergehenden Ansprüche, dadurch gekennzeichnet, daß die Einführung der tert.-Alkylgruppen durch Friedel-Crafts-Alkylierung oder nukleophile Substitution erfolgt.

5. Verfahren nach einem der vorhergehenden Ansprüche, dadurch gekennzeichnet, daß man als Ausgangsverbindungen Naphthalin, Anthracen, Phenanthren, Chrysen, 1,2-Benzanthracen, Decacyclen, Hexahelicen, Tetracen, Diphenyl, Fluoren, 1,12-Benzoperylen, 1,12-o-Phenylenperylen, Coronen, 1,2-Benzocoronen, Bisanthen, 1,4-Benzobisanthen, Ovalen, Circumanthracen, Pyren, Perylen, 1,2,8,9-Dibenzoperopyren, 1,2,9,10-Dibenzoperopyren, Dipyreno-(1',3':10,2),(1",3":5,7)-Pyren, Fluoranthen, 2,3-o-Phenylenpyren, 4,5-o-Phenylenfluoranthen, Isorubicen, Rubicen, 2,3,7,8-Di-(o-phenylen)-pyren), Periflanthen, 11,12-peri-Naphthylenfluoranthen, 2,3,6,7-Di-(peri-naphthylen)-naphthalin, 1,2,3,4,5,6,7,8-Tetra-(peri-naphthylen)-anthracen, Indeno-(2',1':1,2)-perinaphthen, 1,2,4,5-Dibenzopentalen oder 2,3-o-Phenylen-4,5-peri-naphthylenpyren verwendet.

6. Verfahren nach einem der vorhergehenden Ansprüche, dadurch gekennzeichnet, daß man:

a) Nitrofarbstoffe
b) Nitrosofarbstoffe
c) Diphenylmethanfarbstoffe
d) Triphenylmethanfarbstoffe
e) Acridinfarbstoffe
f) Xanthenfarbstoffe
g) Azinfarbstoffe
h) Methinfarbstoffe
i) Chinonimine
j) Anthrachinonfarbstoffe
k) Indigoide Farbstoffe
l) Chinoide Küpenfarbstoffe
m) Indigosole
n) Schwefelfarbstoffe
o) Stilbenfarbstoffe
p) Thiazolfarbstoffe
q) Azofarbstoffe
r) Phthalocyanine oder
s) Porphyrine

oder deren Vorstufen als Ausgangsmaterial verwendet.

0039085

Nummer der Anmeldung

EP 81 10 3230

Europäisches Patentamt

# EUROPÄISCHER RECHERCHENBERICHT

| EINSCHLÄGIGE DOKUMENTE | | | KLASSIFIKATION DER ANMELDUNG (Int. Cl.³) |
|---|---|---|---|
| Kategorie | Kennzeichnung des Dokuments mit Angabe, soweit erforderlich, der maßgeblichen Teile | betrifft Anspruch | |
| A | DE - A - 1 794 136 (J.R. GEIGY)<br>* Seite 8; Beispiele * | | C 07 B 29/00<br>27/00<br>C 07 C 13/62<br>C 07 D 471/06 |
| D | & FR - A - 1 580 683<br>-- | | |
| AD | G.A. OLAH: "Friedel-Crafts and Related Reactions", Band II: "Alkylation and Related Reactions" Teil I, 1964, Interscience Publishers<br>New York, London, Sydney<br>* Seite 183 *<br>-- | | RECHERCHIERTE SACHGEBIETE (Int. Cl.³) |
| A | US - A - 2 997 433 (T.D.NEVITT)<br>---- | | C 07 B 29/00<br>27/00<br>C 07 C 13/62<br>2/86<br>C 07 D 471/06<br>C 09 B 43/00<br>5/62<br>47/12 |

KATEGORIE DER GENANNTEN DOKUMENTE

X: von besonderer Bedeutung
A: technologischer Hintergrund
O: nichtschriftliche Offenbarung
P: Zwischenliteratur
T: der Erfindung zugrunde liegende Theorien oder Grundsatze
E: kollidierende Anmeldung
D: in der Anmeldung angefuhrtes Dokument
L: aus andern Gründen angefuhrtes Dokument
&: Mitglied der gleichen Patentfamilie, übereinstimmendes Dokument

X Der vorliegende Recherchenbericht wurde für alle Patentansprüche erstellt.

| Recherchenort | Abschlußdatum der Recherche | Prüfer |
|---|---|---|
| Den Haag | 27-07-1981 | VAN GEYT |